(19) **Europäisches Patentamt**
**European Patent Office**
**Office européen des brevets**

(11) **EP 1 645 225 B1**

(12) **EUROPEAN PATENT SPECIFICATION**

(45) Date of publication and mention
of the grant of the patent:
**19.03.2008 Bulletin 2008/12**

(51) Int Cl.:
***A61B 5/02*** (2006.01)

(21) Application number: **05021664.7**

(22) Date of filing: **04.10.2005**

(54) **Apparatus, program product and method for displaying pulse wave information**

Vorrichtung, Programmprodukt und Verfahren zum Anzeigen von Pulswelleninformation

Dispositif, produit programme et procédé pour afficher d'information d'onde de pouls

(84) Designated Contracting States:
**DE ES FR GB IT NL**

(30) Priority: **05.10.2004 JP 2004292264**

(43) Date of publication of application:
**12.04.2006 Bulletin 2006/15**

(73) Proprietor: **Omron Healthcare Co., Ltd.**
**Kyoto-shi,**
**Kyoto 615-0084 (JP)**

(72) Inventors:
• **Oshiumi, Akira,**
**c/o Omron Healthcare Co, Ltd**
**Kyoto-shi**
**Kyoto 615-0084 (JP)**
• **Inagaki, Takashi,**
**c/o Omron Healthcare Co, Ltd**
**Kyoto-shi**
**Kyoto 615-0084 (JP)**
• **Nishinohira, Yukiko,**
**c/o Omron Healthcare Co, Ltd**
**Kyoto-shi**
**Kyoto 615-0084 (JP)**

(74) Representative: **Kilian, Helmut**
**Wilhelms, Kilian & Partner**
**Patentanwälte**
**Eduard-Schmid-Strasse 2**
**81541 München (DE)**

(56) References cited:
**EP-A- 1 356 766** **EP-A- 1 426 007**
**WO-A-20/04002302** **US-A- 5 807 267**
**US-B1- 6 272 376**

• **NICHOLS W W ET AL: "ARTERIAL ELASTANCE AND WAVE REFLECTION AUGMENTATION OF SYSTOLIC BLOOD PRESSURE: DELETERIOUS EFFECTS AND IMPLICATIONS FOR THERAPY" JOURNAL OF CARDIOVASCULAR PHARMACOLOGY AND THERAPEUTICS, CHURCHILL LIVINGSTONE, NAPERVILE, IL, US, vol. 6, no. 1, January 2001 (2001-01), pages 5-21, XP008028712 ISSN: 1074-2484**

**Description**

BACKGROUND OF THE INVENTION

Field of the Invention

The present invention relates to a pulse wave information display apparatus, a program product for controlling the pulse wave information display apparatus, and a method of displaying pulse wave information. More particularly, the present invention relates to a pulse wave information display apparatus, a program product for controlling the pulse wave information display apparatus, and a method of displaying pulse wave information for successively displaying a waveform of one pulse from measured pulse wave data.

Description of the Background Art

Blood pressure is the pressure exerted against the internal walls of the arteries by blood flow generated by contraction and expansion of the heart, and consists of systolic pressure that is the blood pressure in a systolic phase of the heart and diastolic pressure that is the blood pressure in a diastolic phase of the heart. Pressure pulse wave of the intra-arterial pressure is a composite wave of an early systolic component (ejected wave) generated by ejection of blood from the heart and a late systolic component (reflected wave) generated by reflection mainly from the arteries.

Prior art document 1 (Japanese Patent Laying-Open No. 7-39530) and prior art document 2 (the specification of US Patent No. 5265011) disclose a method of extracting characteristic points such as peak points of the ejected wave or the reflected wave described above from measured pulse wave data. Prior art document 1 discloses a method of calculating characteristic points by using zero crossing points of a fourth derivative wave, while prior art document 2 discloses a method of using peaks of a first derivative wave to detect a waveform of one beat and using local maxima and zero crossing points of a third derivative wave, and local maxima of an original waveform, to calculate characteristic points.

Prior art document 3 (Japanese Patent Laying-Open No. 2004-195204 corresponding to EP 1 426 007 A), forming the basis for the preamble of claims 1 and 13, discloses an apparatus that calculates characteristic points from measured pulse wave data to calculate a characteristic value such as an Augmentation Index (AI) and display the same.

The AI is a characteristic value that reflects a time phase and a reflection intensity of a pulse wave, which mainly correspond to the arterial sclerosis. The AI is expressed in index form. The AI is said to be an effective index for the early detection of the circulatory system diseases, in particular, and is known to exhibit a behavior different from that of blood pressure.

Generally, an ejected wave tends to have a higher peak than a reflected wave in younger subjects, whereas a reflected wave tends to have a higher peak than an ejected wave in elder subjects. This is because since elder subjects develop sclerosis of the inner walls of the blood vessels (arterial sclerosis), an ejected wave cannot be fully absorbed by the blood vessel walls, resulting in that a higher level of reflection is detected within a short period of time. As such, the AI value is a value obtained from an ejected wave and a reflected wave both generated by a cardiac beat, and is a characteristic value reflecting a time phase and a reflection intensity of a pulse wave, which mainly correspond to the arterial sclerosis.

Moreover, a pulse wave measurement apparatus "SphygmoCor" is available from Atcor Medical Pty. Ltd. (prior art document 4: a home page of Atcor Medical Pty. Ltd. (updated on February 9, 2004) [retrieved on September 13, 2004], see an internet site <URL: http://www.atcormedical.com/pdf/prodpx.pdf>). This product can display the results of an analysis of measured pulse wave data.

On a display screen of the apparatus disclosed in each of prior art document 3 and prior art document 4, a waveform of one beat in a pulse wave is displayed on an enlarged scale.

However, a waveform displayed on a display screen of each of the documents is only a waveform cut out to correspond to one beat and displayed in succession. Therefore, a waveform of one beat is displayed in a graph where the unit of the ordinate axis is an intravascular pressure value, which is an absolute value. In this case, if one peak point, namely, a reference value for an AI value changes, the height of the peak point changes. Therefore, the change in AI value, which is to be primarily observed, cannot be grasped intuitively as the height of the other peak point.

SUMMARY OF THE INVENTION

The present invention has been made in consideration of the aforementioned problem. An object of the invention is to provide a pulse wave information display apparatus, a program product for controlling the pulse wave information display apparatus, and a method of displaying pulse wave information, by which the change in AI value can intuitively be grasped.

A pulse wave information display apparatus according to one aspect of the present invention as defined in claim 1 includes: a storage portion; an extraction portion; a specification portion; a normalization portion; a plot portion; and a display portion. The storage portion stores measured pulse wave data. The extraction portion extracts a waveform of one beat from the stored pulse wave data. The specification portion specifies a first characteristic point corresponding to a global maximum of an early systolic component (ejected wave) in the extracted waveform of one beat. The normalization portion normalizes the waveform of one beat based on an amplitude value at the specified first characteristic point. The plot portion generates a signal for plotting the normalized waveform of one beat. The display portion displays accordingly based on an output from the plot portion.

The "amplitude value" represents a pressure difference between a pressure value at each of the points that constitute a waveform of one beat and a pressure value at a rising point (starting point) of the waveform.

Preferably, the specification portion further specifies a second characteristic point corresponding to a global maximum of a late systolic component in the extracted waveform of one beat, and the pulse wave information display apparatus further includes a marker display portion for displaying markers on the display portion to indicate a position corresponding to the first characteristic point and a position corresponding to the second characteristic point in the normalized waveform of one beat.

It is desirable that the pulse wave information display apparatus further includes a pulse wave measurement portion for measuring the pulse wave data.

Preferably, the specification portion further specifies a second characteristic point corresponding to a global maximum of a late systolic component in the extracted waveform of one beat, and the pulse wave information display apparatus further includes a ratio display portion for simultaneously displaying on the display portion a ratio of an amplitude value at the second characteristic point to an amplitude value at the first characteristic point, and the normalized waveform of one beat.

It is desirable that the pulse wave information display apparatus displays on the display portion a waveform based on a user's instruction and the normalized waveform in a superimposed manner.

Preferably, the specification portion further specifies a second characteristic point corresponding to a global maximum of a late systolic component in the extracted waveform of one beat, and the pulse wave information display apparatus further includes a determination portion for determining whether or not a value at a point corresponding to the second characteristic point in the normalized waveform exceeds a displayable upper limit value, and an update portion for updating the upper limit value based on a result of the determination by the determination portion such that the value at the point corresponding to the second characteristic point can be displayed.

Furthermore, in the aforementioned pulse wave information display apparatus, the characteristic point is desirably extracted as follows for specification:

The specification portion extracts the first characteristic point based on local maxima of a fourth derivative wave of a pulse wave of one beat, and specifies the same.

Alternatively, the specification portion extracts the first characteristic point based on zero crossing points of the fourth derivative wave of the pulse wave of one beat, and specifies the same.

Furthermore, the specification portion extracts the second characteristic point based on the local maxima of the fourth derivative wave of the pulse wave of one beat, and specifies the same.

Alternatively, the specification portion extracts the second characteristic point based on the zero crossing points of the fourth derivative wave of the pulse wave of one beat, and specifies the same.

A computer-readable medium as defined in claim 9 is a program product for controlling a pulse wave information display apparatus including a storage portion for storing pulse wave data, an arithmetic processing portion for arithmetically processing the pulse wave data stored in the storage portion, and a display portion. The program product allows the arithmetic processing portion to perform the steps of: extracting a waveform of one beat from the pulse wave data stored in the storage portion; specifying a first characteristic point corresponding to a global maximum of an early systolic component in the extracted waveform of one beat; normalizing the waveform of one beat based on an amplitude value at the specified first characteristic point; and displaying the normalized waveform of one beat on the display portion.

Preferably, the step of specifying includes the step of specifying a second characteristic point corresponding to a global maximum of a late systolic component in the extracted waveform of one beat, and the program product further allows the arithmetic processing portion to perform the step of displaying markers on the display portion to indicate a position corresponding to the first characteristic point and a position corresponding to the second characteristic point in the normalized waveform of one beat.

Preferably, the step of specifying includes the step of specifying a second characteristic point corresponding to a global maximum of a late systolic component in the extracted waveform of one beat, and the program product further allows the arithmetic processing portion to perform the step of simultaneously displaying on the display portion a ratio of an amplitude value at the second characteristic point to an amplitude value at the first characteristic point, and the normalized waveform of one beat.

Preferably, the step of specifying includes the step of specifying a second characteristic point corresponding to a global maximum of a late systolic component in the extracted waveform of one beat, and the program product further allows the arithmetic processing portion to perform the steps of determining whether or not a value at a point corresponding to the second characteristic point in the normalized waveform exceeds a displayable upper limit value, and updating the upper limit value based on a result of the determination such that the value at the point corresponding to the second characteristic point can be displayed.

A method of displaying pulse wave information according to still another aspect of the present invention as defined in claim 13 is a method of displaying pulse wave information in a pulse wave information display apparatus including a storage portion for storing pulse wave data, and a display portion for displaying information based on the pulse wave data. The method includes the steps of: extracting a waveform of one beat from the pulse wave data stored in the storage portion; specifying a first characteristic point corresponding to a global maximum of an early systolic component in the extracted waveform of one beat; normalizing the waveform of one beat based on an amplitude value at the specified first characteristic point; and displaying the normalized waveform of one beat on the display portion.

According to the present invention, a normalized waveform is displayed based on an amplitude value at the first characteristic point corresponding to a global maximum of an early systolic component. Therefore, a user can intuitively grasp the change in AI value.

The foregoing and other objects, features, aspects and advantages of the present invention will become more apparent from the following detailed description of the present invention when taken in conjunction with the accompanying drawings.

BRIEF DESCRIPTION OF THE DRAWINGS

Fig. 1 shows a specific example of a configuration of a pulse wave information display apparatus according to first and second embodiments of the present invention.

Fig. 2 is a flowchart showing a flow of display processing for pulse wave information according to the first and second embodiments of the present invention.

Fig. 3 shows an example of pulse wave information displayed on a display portion of the pulse wave information display apparatus according to the first and second embodiments of the present invention.

Fig. 4 shows a specific example in the case where a waveform of one beat is displayed by using an absolute value.

Fig. 5 shows a specific example in the case where a waveform of one beat is displayed by using a ratio.

Fig. 6 is a flowchart showing a flow of display processing according to the first embodiment of the present invention.

Fig. 7 shows an example of how a normalized waveform is displayed.

Fig. 8 is a flowchart showing a flow of display processing according to the second embodiment of the present invention.

Fig. 9 is a flowchart showing a flow of adjusting a vertical range according to the second embodiment of the present invention.

Fig. 10 shows a specific example in the case where the upper limit value of the vertical range is adjusted.

Fig. 11 is a first view for describing an AI value.

Fig. 12 is a second view for describing the AI value.

Fig. 13 is a view for describing processing of extracting a characteristic point.

DESCRIPTION OF THE PREFERRED EMBODIMENTS

Embodiments of the present invention will hereinafter be described in detail with reference to the drawings. In the drawings, like components are denoted by like reference characters and the description thereof will not be repeated.

First Embodiment

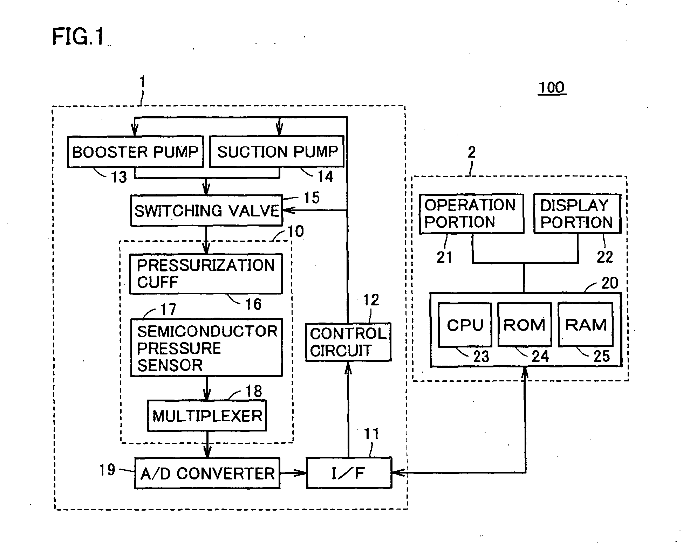

Fig. 1 shows a specific example of a configuration of a sphygmograph 100 according to a first embodiment of the present invention. Referring to Fig. 1, sphygmograph 100 includes a pulse wave measurement portion 1 for measuring a pulse wave, and a pulse wave information display apparatus 2. Pulse wave measurement portion 1 and pulse wave information display apparatus 2 are connected via a dedicated cable such as a Universal Serial Bus (USB), or a communication line. It is noted that the connection also includes a non-contact communication such as a radio communication.

Pulse wave information display apparatus 2 includes a processing portion 20 that has a Read Only Memory (ROM) 24 and a Random Access Memory (RAM) 25 for storing data and a program for controlling sphygmograph 100, and a Central Processing Unit (CPU) 23 for controlling the whole sphygmograph 100, an operation portion 21 that is provided to be operable from the outside and is accordingly operated for entering various types of information, and a display portion 22 comprised of, for example, a Light Emitting Diode (LED) and a Liquid Crystal Display (LCD) for outputting various types of information such as the results of the pulse wave measurement.

CPU 23 accesses ROM 24 to read a program and expand the program on RAM 25 for execution of the program to control the whole sphygmograph 100. Furthermore, CPU 23 receives from operation portion 21 an operation signal from a user and performs control processing for the whole sphygmograph 100 based on the operation signal. Furthermore, CPU 23 provides control such that information of pulse wave data obtained from pulse wave measurement portion 1 and the like is displayed on display portion 22. The first embodiment is allowed to have a configuration shown in Fig. 1, thereby CPU 23 in pulse wave information display apparatus 2 can display, on display portion 22 in real time, the pulse wave data measured by pulse wave measurement portion 1.

A generally-used Personal Computer (PC), for example, may serve as pulse wave information display apparatus 2. The configuration of pulse wave information display apparatus 2 shown in Fig. 1 is a specific example of a configuration of a generally-used PC. Therefore, the configuration of pulse wave information display apparatus 2 is not limited to that shown in Fig. 1.

Pulse wave measurement portion 1 receives a control signal from pulse wave information display apparatus 2 via an I/F 11. The control signal received at I/F 11 is then sent to a control circuit 12, from which the control signal is sent to a booster pump 13, a suction pump 14, or a switching valve 15.

Booster pump 13 is a pump for boosting the internal pressure (hereafter referred to as "cuff pressure") of a pressurization cuff (air bag) 16, and suction pump 14 is a pump for decreasing the cuff pressure. Switching valve 15 selectively switches and connects either of booster pump 13 or suction pump 14 to an air tube (not shown). Control circuit 12 controls these components.

A semiconductor pressure sensor 17 is configured to have a semiconductor chip made of single crystal silicon or the like and having a plurality of sensor elements arranged thereon in one direction at predetermined intervals. Semiconductor pressure sensor 17 is pressed, with the pressure of pressurization cuff 16, against a measurement site such as a wrist of a subject undergoing the measurement. In this state, semiconductor pressure sensor 17 detects a pulse wave of the subject via the radial artery. A voltage signal obtained by detecting a pulse wave in semiconductor pressure sensor 17 is amplified in an amplifier 18 to be output to an A/D converter 19. A/D converter 19 converts the voltage signal, which is an analog signal derived from semiconductor pressure sensor 17 through amplifier 18, into digital information. The digital information is sent out to pulse wave information display apparatus 2 via I/F 11

The digital information sent out via I/F 11, namely, pulse wave data; is sequentially stored in RAM 25 and the like. CPU 23 reads the stored digital information in real time to perform various types of arithmetic processing.

In Fig. 1, pulse wave measurement portion 1 and pulse wave information display apparatus 2 are shown separately. However, they may be configured integrally.

Pulse wave information display apparatus 2 according to the first embodiment is shown to analyze the pulse wave data measured by pulse wave measurement portion 1 and display the same in real time. However, the pulse wave information display apparatus is not limited as such. For example, the pulse wave data measured in pulse wave measurement portion 1 may be stored in RAM 25 or on a hard disk not shown to be displayed later. Furthermore, the pulse wave data measured in pulse wave measurement portion 1 may be stored in a memory card not shown, so that the memory card is inserted into pulse wave information display apparatus 2, thereby CPU 23 may perform arithmetic processing for display as described below.

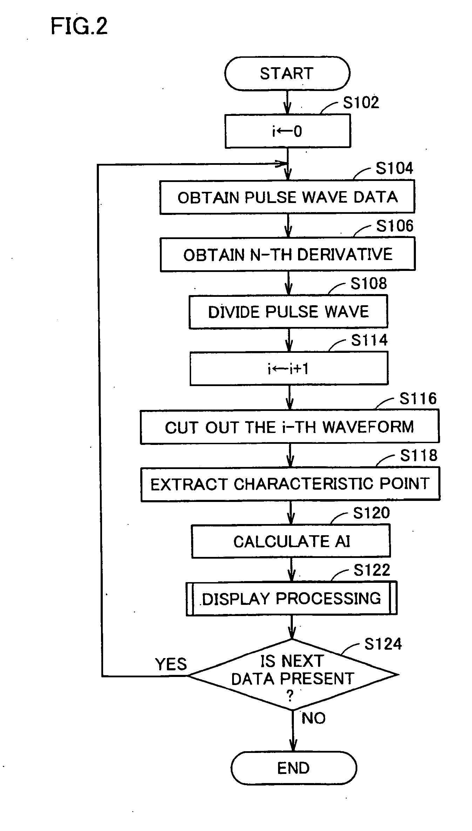

Display processing for the pulse wave information in the pulse wave information display apparatus 2 according to the present embodiment will now be described with reference to a flowchart shown in Fig. 2. CPU 23 in pulse wave information display apparatus 2 accesses ROM 24 for reading a program and expands the program on RAM 25 for execution, thereby the processing shown in the flowchart of Fig. 2 is implemented.

A drive apparatus (not shown) provided in pulse wave information display apparatus 2 may read a program recorded on a recording medium so that the program may be expanded on RAM 25 for execution.

Referring to Fig. 2, initially, a variable i is initialized into "0" (step S (hereinafter simply referred to as "S") 102). In the present embodiment, variable i is a symbol for representing the order of waveforms each corresponding to one beat extracted from pulse wave data. CPU 23 obtains pulse wave data (step S104). In the present embodiment, the pulse wave data, which is a digital signal obtained via I/F 11 in pulse wave measurement portion 1, is sequentially obtained from RAM 25.

A program stored in ROM 24 is executed to obtain N-th derivatives of a pulse waveform derived from the pulse wave data (S106). Based on a result of the differentiation, the pulse waveform is divided into pulse waves each corresponding to one beat (S 108).

The processing of dividing the pulse wave in step S 108 is performed, for example, as described below. Specifically, a negative to positive transition of the first derivative among the N-th derivatives obtained in step S 106 is waited for. When the first derivative changes from negative to positive, the rising zero crossing point (point DZC) is held and a point that is on the original waveform and in the same time phase as that of point DZC is defined as a "temporary rising point (point PB)". Then a local maximum (point DP) of the first derivative is waited for. When the local maximum of the first derivative is sensed, CPU 23 determines whether one beat can be confirmed or not. Specifically, a local maximum value (point PQ) of the pulse waveform (original waveform) is waited for and sensed just after the temporary rising point (point PB). In response to the detection of the local maximum value (point PQ), CPU 23 refers to the waveform from point PB to another temporary rising point (point PA) of a preceding beat. It is then confirmed that the global maximum (point PP) of the pulse waveform (original waveform) is present between point PA and point PB, and that point PB is the global minimum of the waveform between point PP and point PQ. Point PB is thus confirmed as the global minimum and accordingly point PB is defined as a "rising point". The waveform from point PA to point PB is thus the waveform of one beat.

CPU 23 then increments variable i by "1" (S 114). A waveform of one beat, which has been obtained by the division in step S108, is designated as a variable i-th waveform, and the variable i-th waveform is cut out (S116). From the variable i-th waveform cut out in step S 116, prescribed characteristic points are extracted (S118).

In the present embodiment, CPU 23 extracts a rising point (point PA), an ejected wave peak point (also referred to as point P1), and a reflected wave peak point (also referred to as point P2) of the variable i-th waveform.

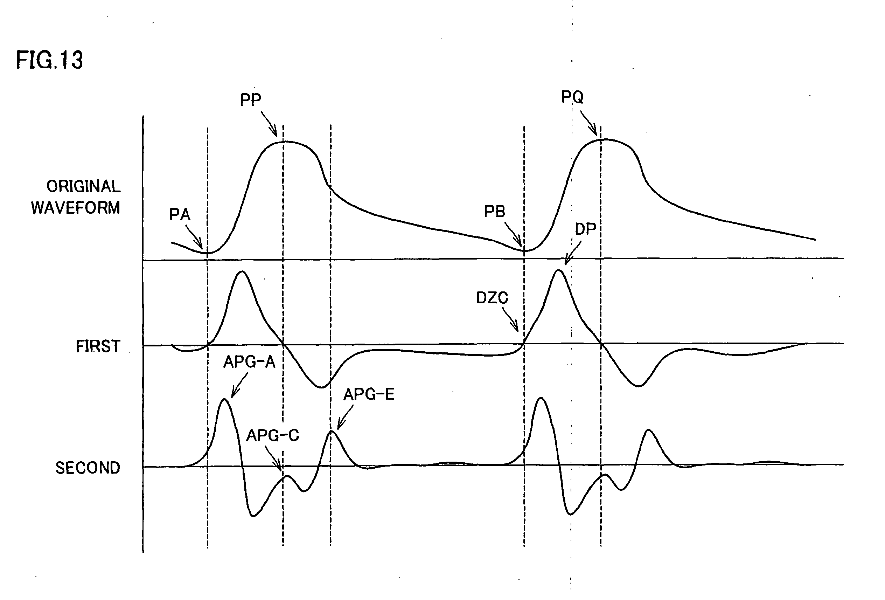

In the present embodiment, point P1 and point P2 are extracted by, for example, using local maxima of a fourth derivative wave of a waveform of one beat. Specifically, referring to Fig. 13, these characteristic points are extracted as described below. Fig. 13 is a part of the drawing described in prior art document 1, to which reference characters are added.

Initially, the waveform of one beat is confirmed and then local maxima of the second derivative between point PA and point PB are obtained. The local maxima of the second derivative obtained here are referred to as a point APG-A, a point APG-C, and a point APG-E in order. Then, local maxima of the fourth derivative between point PA and point APG-E are obtained. The obtained local maxima of the fourth derivative are candidates for a point P1 and a point P2.

Then, among local maxima of the fourth derivative that are present in the section of the ascending limb between point PA and point PP, the maximum one is obtained as point P1. Among local maxima of the forth derivative that are present in the section of the descending limb between point PP and point APG-E, the maximum one is obtained as point P2.

A method of extracting point P1 and point P2 is not limited the above-described method; point P1 and point P2 may be extracted by the methods disclosed in, for example, prior art documents 1 and 2.

CPU 23 then calculates an AI value from the characteristic points extracted in step S 118 (S120). In the present embodiment, the AI value is calculated in accordance with the following expression.

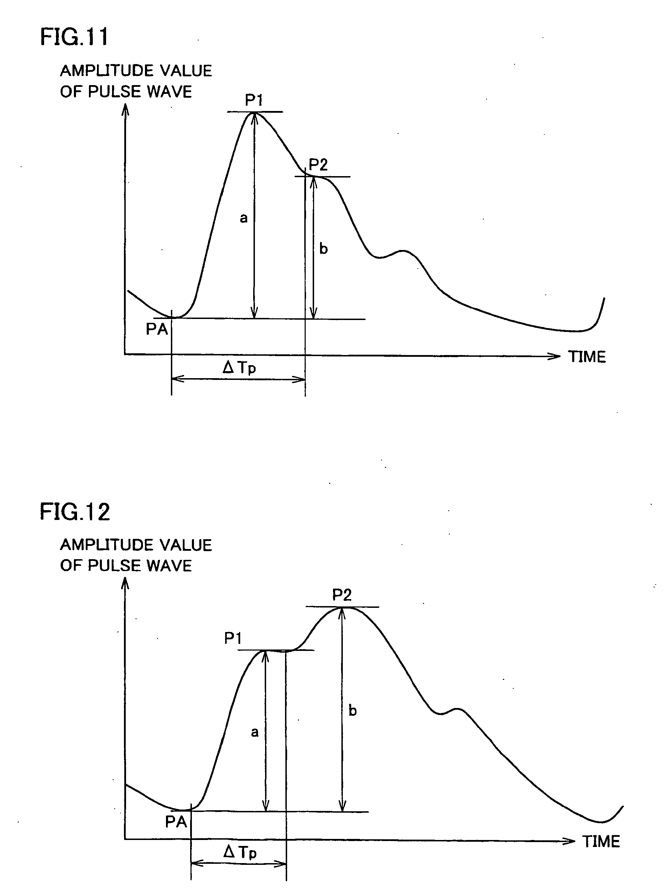

Figs. 11 and 12 show a specific example of the change in intravascular pressure over time in a pulse waveform of one beat. Referring to Figs. 11 and 12, point PA represents a rising point, point P1 represents an ejected wave peak point, and point P2 represents a reflected wave peak point. Given that an amplitude value of the ejected wave (a pressure value at point P1 - a pressure value at point PA) is "a", and that an amplitude value of the reflected wave (a pressure value at point P2 - a pressure value at point PA) is "b", AI (%) is calculated as "(b/a) x 100".

In the present embodiment, a value other than the AI value, for example, $\Delta$Tp (the time at a reflected wave rising point - the time at point PA) shown in Figs. 11 and 12, may further be calculated as a characteristic value. The aforementioned $\Delta$Tp is also a known index just as AI.

CPU 23 then performs display processing described below (S 122). Thereafter, CPU 23 determines whether or not additional pulse wave data is present (S 124). If CPU 23 determines that additional pulse wave data is present (YES in S124), it repeats processing in steps S104-S122. If CPU 23 determines that no additional pulse wave data is present (NO in S124), it terminates the display processing for pulse wave information.

Although every waveform corresponding to one beat is displayed in the first embodiment of the present invention, a waveform corresponding to two or three beats may be displayed. Alternatively, a waveform corresponding to a predetermined number of beats may not be displayed; a waveform of one beat may be updated successively and displayed.

It is noted that the specification of characteristic points includes the extraction of characteristic points from the waveform data of one beat, as shown in step S 118, as well as, if data concerning the extracted characteristic points is included in the pulse wave data, the specification of the characteristic points based on the data.

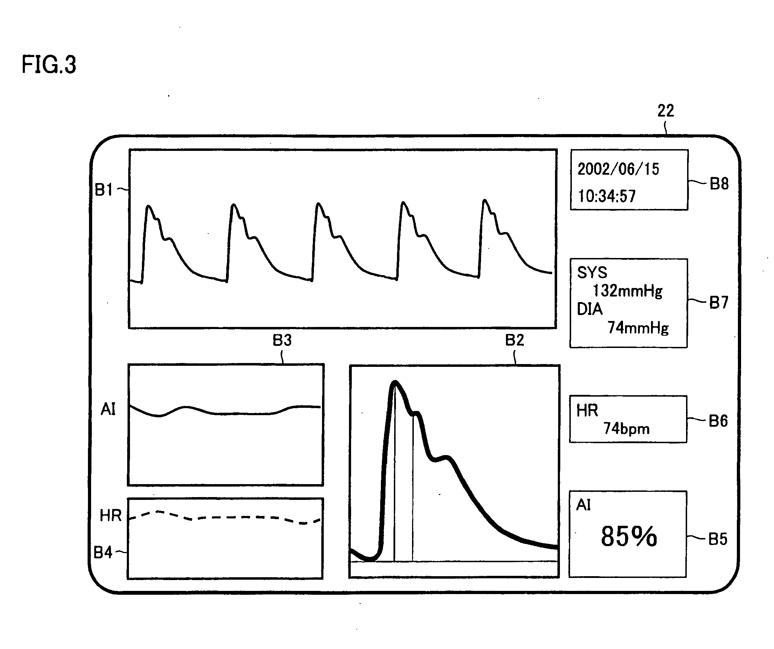

Fig. 3 shows a specific example of pulse wave information displayed on display portion 22 of pulse wave information display apparatus 2 in the present embodiment.

Referring to Fig. 3, pulse wave data obtained by measurement is displayed in a display region B 1 in real time. Furthermore, a waveform of one beat, which has been obtained through the division in step S108, is successively displayed in a display region B2.

The change in AI value over time, which has been calculated in step S 115, is displayed in a display region B3. A change in heart rate HR over time is displayed in a display region B4. At the same time, an AI value (%) of the waveform of one beat displayed in display region B2 is displayed in a display region B5. Moreover, the average of heart rate HR is displayed in a display region B6. Systolic blood pressure SYS and diastolic blood pressure DIA are displayed in a display region B7. The date and time of measurement are displayed in a display region B8. The time-variable information displayed here is displayed by plotting a measured value in relation to the measurement time counted by a clock not shown.

In the present embodiment, the AI value of a waveform of one beat is displayed in real time in display region B5. Accordingly, a change that cannot be recognized only from a blood pressure value can be grasped. In particular, when a fast-acting antihypertensive agent is required to be administered as an emergency treatment of abnormal hypertension during surgery or in the case of hypertensive emergency, acute heart failure, and the like, it is possible to recognize the antihypertensive effect, which cannot be recognized only from a blood pressure value. Therefore, the present embodiment is effective in improving the accuracy with which whether a proper amount of the agent is administered or not is determined, or in reducing time required for the determination.

Furthermore, a waveform of one beat is successively displayed in display region B2 in real time. As such, since a waveform of one beat is displayed in display region B2 on an enlarged scale, the shape of the waveform of one beat can be viewed in more detail.

In display region B2, a reference waveform based on a user's instruction and the aforementioned waveform of one beat may be displayed in a superimposed manner. In this case, the reference waveform can be distinguished from the waveform of one beat by, for example, displaying both of them in different colors. The reference waveform may be registered as follows: a user operates operation portion 21 to specify a waveform at a certain time point from the waveforms successively displayed, and registers the same. Alternatively, a user may operate operation portion 21 to register an average waveform from one time point to another time point. As such, by displaying the reference waveform and the waveform of one beat in a superimposed manner, it is much easier to visually confirm the effect before and after the administration of an agent.

Furthermore, the above-described ΔTp may be displayed at the same time.

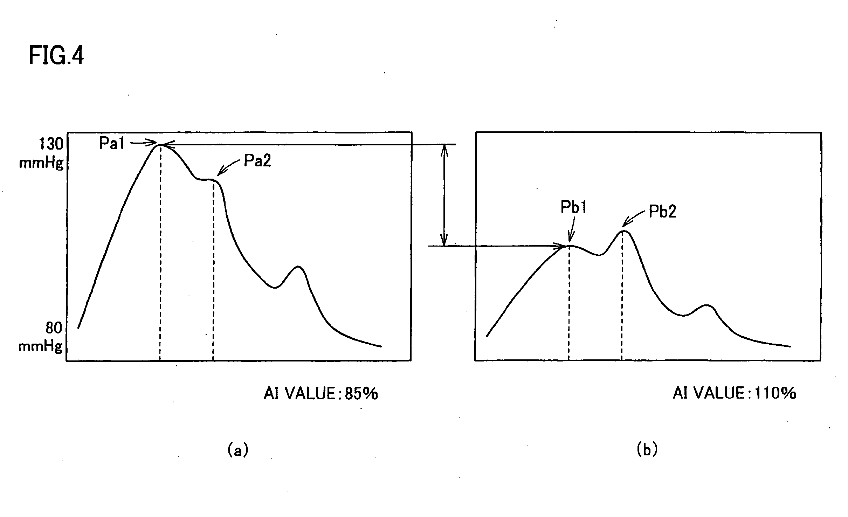

Here, a waveform of one beat displayed in display region B2 is generally displayed in a graph where the units of the abscissa axis and the ordinate axis are time and pressure value (unit: mmHg), respectively, as shown in Fig. 4. Fig. 4 shows that absolute values of 80 mmHg-130 mmHg are marked on the ordinate axis.

The waveform shown in Fig. 4(a) has a pressure value at ejected wave peak point Pa1 of approximately 125 mmHg, and an amplitude value of approximately 45 mmHg. An AI value of the waveform shown in Fig. 4(a) is 85%.

In contrast, the waveform shown in Fig. 4(b) has a pressure value at ejected wave peak point Pb1 of approximately 105 mmHg, and an amplitude value of approximately 25 mmHg. An AI value of the waveform shown in Fig. 4 (b) is 110%.

As such, although the waveform shown in Fig. 4(b) has a higher AI value, ejected wave peak point Pa1 shown in Fig. 4(a) has an amplitude value larger than that of ejected wave peak point Pb1 shown in Fig. 4(b). Therefore, reflected wave peak point Pa2 shown in Fig. 4(a) is displayed to have a height larger than that of reflected wave peak point Pb2 shown in Fig. 4(b). Consequently, even if a waveform corresponding to one beat is successively displayed in display region B2 of display portion 22, the change in AI value cannot be grasped visually.

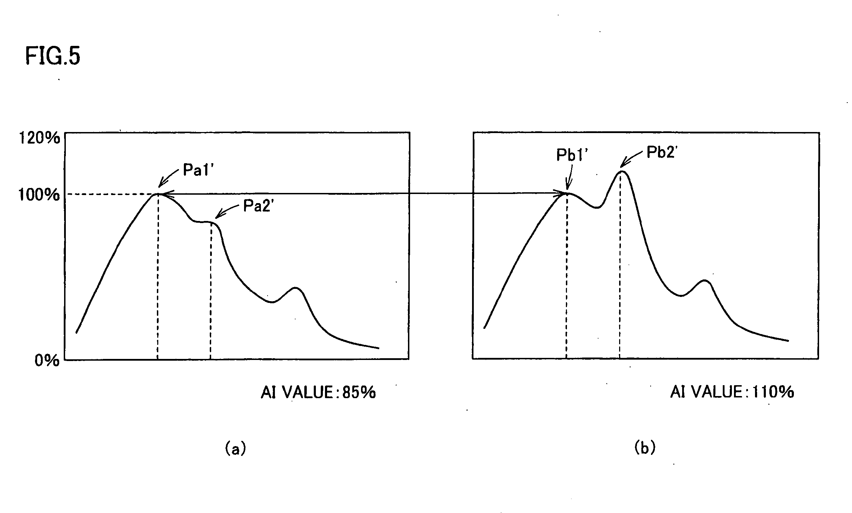

Therefore, Fig. 5 shows specific examples in which amplitude values at the ejected wave peak points, each of which is a reference (denominator) for an AI value, are set at a certain level for display.

Referring to Fig. 5, the specific examples are shown in a graph where the unit of the abscissa axis is time as in Fig. 4, while the unit of the ordinate axis is ratio (%) with respect to the amplitude value at the ejected wave peak point. Fig. 5 shows that ratios of 0%-120% are marked on the ordinate axis. The waveform shown in Fig. 5(a) is a waveform corresponding to that of Fig. 4(a), while the waveform shown in Fig. 5(b) is a waveform corresponding to that of Fig. 4(b).

Each of the heights of ejected wave peak point Pa1' shown in Fig. 5(a) and ejected wave peak point Pb1' shown in Fig. 5(b) is shown at a position of 100%. In this case, since the height of reflected wave peak point Pa2' shown in Fig. 5(a) is described by "(an amplitude value at point Pa2') / (an amplitude value at point Pa1') x 100", it is displayed at a position of 85%, which is equal to the AI value. Since the height of reflected wave peak point Pb2' shown in Fig. 5 (b) is described by "(an amplitude value at point Pb2') / (an amplitude value at point Pb1') x 100", it is displayed at a position of 110%, which is equal to the AI value.

As such, by normalizing a waveform of one beat with respect to an amplitude value at the ejected wave peak point, an increase/decrease in AI value can be recognized in a graph through the fluctuations of the reflected wave peak point in the waveform. Therefore, a user can more intuitively grasp the change in AI value than in the case where the change in AI value is indicated by a numeric value. Since the change in AI value is not affected by an absolute value of the pressure, the degree of reflection of a pressure wave can accurately be grasped.

As described above, by observing a waveform of one beat that has been normalized with respect to an amplitude value at the ejected wave peak point, it is possible to grasp the change in vessels due to an antihypertensive agent as the change in reflected wave peak point, without being affected by the change in blood pressure (= the change in absolute value of the pressure). Therefore, by illustrating such a normalized waveform successively, the effect of an antihypertensive agent can be observed in detail, which is thought to be useful in determining whether the antihypertensive agent should further be administered, or whether an alternative agent should be used.

Therefore, in the first embodiment of the present invention, a waveform normalized with respect to an amplitude value at the ejected wave peak point is sequentially displayed in display region B2 of display portion 22 as shown in Fig. 5.

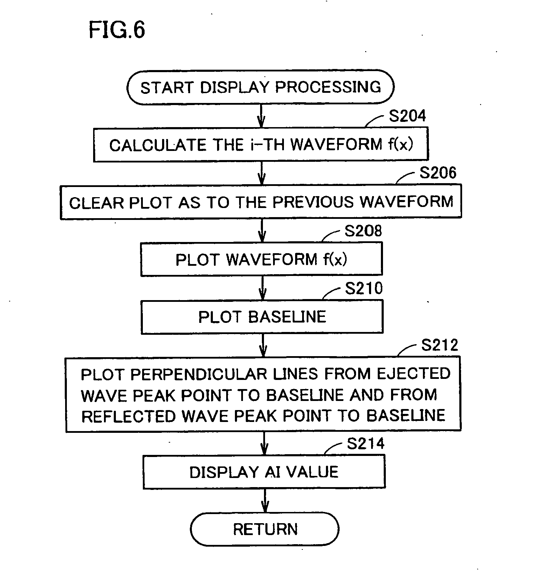

Here, a flow of display processing in step S122 of Fig. 2 will now be described with reference to a flowchart shown in Fig. 6. CPU 23 accesses ROM 24 to read a program and expands the program on RAM 25 for execution, thereby the processing shown in the flowchart in Fig. 6 is also implemented.

Referring to Fig. 6, CPU 23 of pulse wave information display apparatus 2 initially normalizes the i-th waveform, in other words, calculates a waveform f(x) in accordance with the following expression (S204).

$$f(x) = 100 \times \{g(x) - g(x_0)\} / \{g(x_1) - g(x_0)\}$$

In the expression above, "g(x)" represents an original waveform, while "f(x)" represents a normalized waveform. "$x_0$" represents time at a rising point (PA), while "$x_1$" represents time at an ejected wave peak point (P1).

With the expression above, the unit of the ordinate axis in display region B2 of display portion 22 is converted from "a pressure value" into "a ratio" based on an amplitude value at ejected wave peak point P1, and original waveform g(x) is normalized. Assume that waveform f(x) normalized in such a manner has a rising point PA', an ejected wave peak point P1', a reflected wave peak point P2', and an endpoint of the waveform PB'.

When the i-th normalized waveform f(x) is calculated in step S204, the plot of the previous waveform, namely, the (i - 1) th waveform is cleared (S206).

Waveform f(x) from rising point PA to endpoint PB' is then plotted in display region B2 of display portion 22 (S208). In the first embodiment, the display range is fixed, and thus CPU 23 plots the normalized waveform f(x) based on the predetermined upper limit value (e.g. 120%) of the ordinate axis. A value that the value at reflected wave peak point P2' is not assumed to exceed under normal conditions may be set as the upper limit value. Alternatively, a user may operate operation portion 21 to set the upper limit value.

It is sufficient for the waveform of one beat displayed in display region B2 to include points corresponding to at least rising point PA' through a dicrotic notch point (above-described "point APG-E").

CPU 23 then plots a baseline L0 horizontally with respect to the height of rising point PA', namely, the height of 0% (S210). Furthermore, CPU 23 plots a perpendicular line L 1 from ejected wave peak point P1' to the baseline, and a perpendicular line L2 from reflected wave peak point P2' to the baseline (S212).

In steps S210 and S 212, rising point PA', ejected wave peak point P1', and reflected wave peak point P2' are indicated by straight lines as markers. However, how these ponts are displayed is not limited thereto. For example, each of the characteristic points may be indicated by an arrow, or these characteristic points may be indicated by different colors.

If the ordinate axis in the display range of display region B2 of display portion 22 has the lower limit value of 0%, baseline L0 may not necessarily be plotted.

CPU 23 then displays the AI value calculated in step S 120 in Fig. 2 as a text in display region B5 of display portion 22 (S214). When the processing above is completed, the process proceeds to step S124 of Fig. 2.

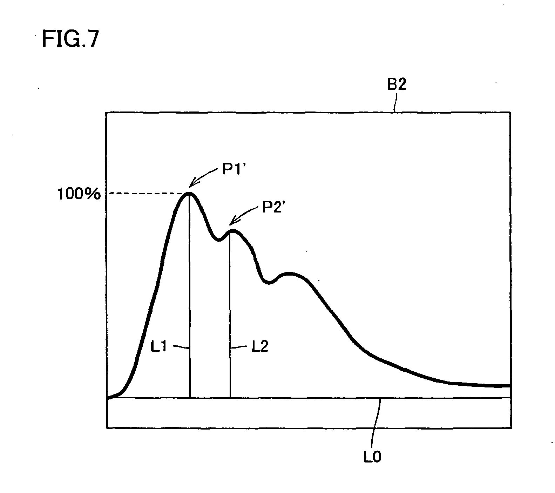

Here, Fig. 7 shows an example in which waveform f(x), baseline L0, and perpendicular lines L1 and L2 are displayed in display region B2 of display portion 22 in the above-described steps S208-S212.

As described above, by displaying perpendicular lines L1 and L2 to allow the positions of ejected wave peak point P1' and reflected wave peak point P2' to be viewable, what AI value means can be recognized in more detail. The AI value has such a characteristic that it increases as the propagation velocity of a pressure wave increases, and that it increases as the intensity of a reflection in the peripheries increases. Therefore, if the AI value is high, the recognition of the positional relation between ejected wave peak point P1' and reflected wave peak point P2' makes it possible to visually understand why the AI value is high: the high AI value is caused by high propagation velocity of a pressure wave, by high reflection intensity in the peripheries, or the like. Accordingly, by checking the AI value against the characteristic of an antihypertensive agent used for treatment, the effect of the antihypertensive agent can be recognized in detail, which is thought to be useful in determining whether the antihypertensive agent should further be administered, or whether an alternative agent should be used.

In the first embodiment, an algorithm for calculating characteristic points for display processing may be an algorithm simpler than that for calculating AI. Therefore, the display processing (S122) is not necessarily performed after the AI calculation (S120) as in the case shown in Fig. 2. Instead, these steps may be performed in parallel.

Second Embodiment

Sphygmograph 100 according to a second embodiment of the present invention will now be described. Configurations and fundamental operations of sphygmograph 100 in the second embodiment are similar to those of the first embodiment, and thus the description thereof will not be repeated.

In pulse wave information display apparatus 2 according to the first embodiment, the normalized waveform is displayed based on the predetermined upper limit value. In other words, the upper limit value of the display range is fixed. Therefore, if the normalized waveform exceeds the upper limit value of the ordinate axis of a display range (hereinafter referred to as a "vertical range"), the exceeding part of the waveform cannot be displayed. Therefore, there is a possibility that the position (height) of the reflected wave peak point is not displayed for a long period of time. As a result, the positional relation between the ejected wave peak point and the reflected wave peak point cannot be viewed, which can make it difficult to intuitively recognize the AI value.

Therefore, in the pulse wave information display apparatus according to the second embodiment, the vertical range is adjusted as well.

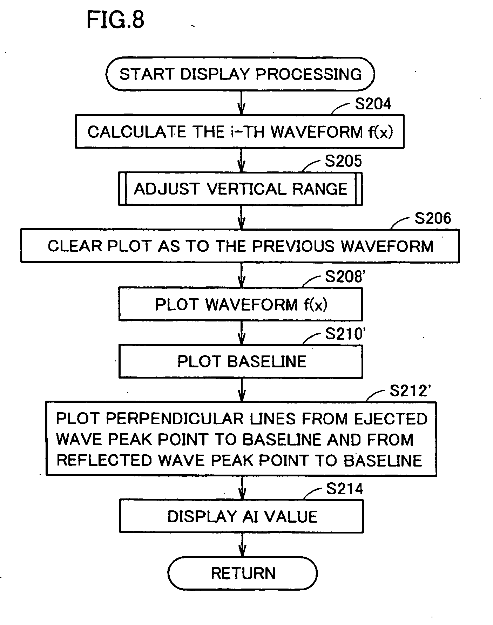

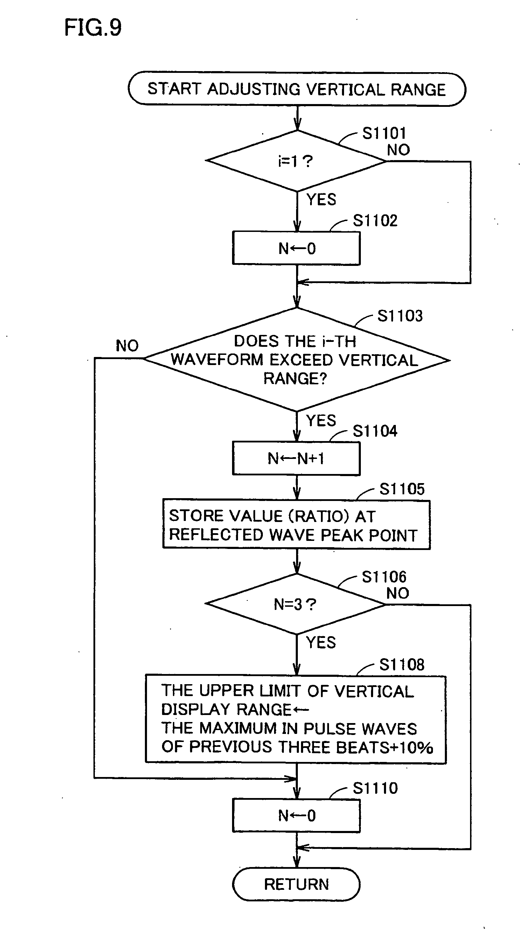

Pulse wave information display processing performed by CPU 23 in pulse wave information display apparatus 2 according to the second embodiment will now be described with reference to flowcharts shown in Figs. 8 and 9. In the display processing shown in Fig. 8, the CPU-performed processing similar to that in Fig. 6 used in the first embodiment is denoted by the same step number. Therefore, the description thereof will not be repeated.

Referring to Fig. 8, a step of adjusting a vertical range (S205) is added between the aforementioned steps S204 and S206 in the display processing according to the second embodiment. Fig. 9 is a flowchart for showing a flow of the step of adjusting a vertical range.

Referring to Fig. 9, CPU 23 initially determines whether variable i is "1" or not, in other words, whether the waveform cut out in step S 116 is the first waveform or not. Since variable i is initially "1" (see step S114 in Fig. 2) (YES in S1101), a count number N is initialized into "0" (S 1102) and the process proceeds to step S 1103.

If CPU 23 determines that variable i is not "1" in step S 1101 (NO in S 1101), the process proceeds to step S 1103.

In step S 1103, CPU 23 determines whether or not a value (ratio (%)) at the reflected wave peak point of the i-th waveform f(x) exceeds the upper limit value (ratio (%)) of the vertical range, which is stored in RAM 25 or the like. If CPU 23 determines that a value at the reflected wave peak point does not exceed the upper limit value (NO in S1103), the process proceeds to step S1110. The initial value of the upper limit value is stored on a hard disk not shown, for example, and transferred to RAM 25 to adjust the vertical range.

In contrast, if CPU 23 determines that a value at the reflected wave peak point exceeds the upper limit value (YES in S1103), then count number N is incremented by "1" (S1104). CPU 23 then allows RAM 25, for example, to store the value (S1105).

CPU 23 then determines whether or not count number N has reached "3", in other words, whether or not the value at the reflected wave peak point is detected to exceed the vertical range three times in succession (S 1106). If CPU 23 determines that count number N has reached "3" (YES in S 1106), a value that equals to the maximum among the values of the previous three beats stored in RAM 25 plus 10%, for example, is set as a new upper limit value of the vertical range for update (S 1108). The process proceeds to step S1110.

If CPU 23 resets count number N in step S1110, the process proceeds to step S206 shown in Fig. 8. If CPU 23 determines in step S 1106 that count number N has not reached "3", in other words, the value at the reflected wave peak point does not exceed the vertical range three times in succession (NO in S 1106), the process proceeds to step S206 shown in Fig. 8.

In steps S208', S210' and S212' in Fig. 8, CPU 23 performs plotting based on the upper limit value stored in RAM 25. Accordingly, if the upper limit value of the vertical range is updated in step S1108, the subsequent display processing is performed based on the updated upper limit value.

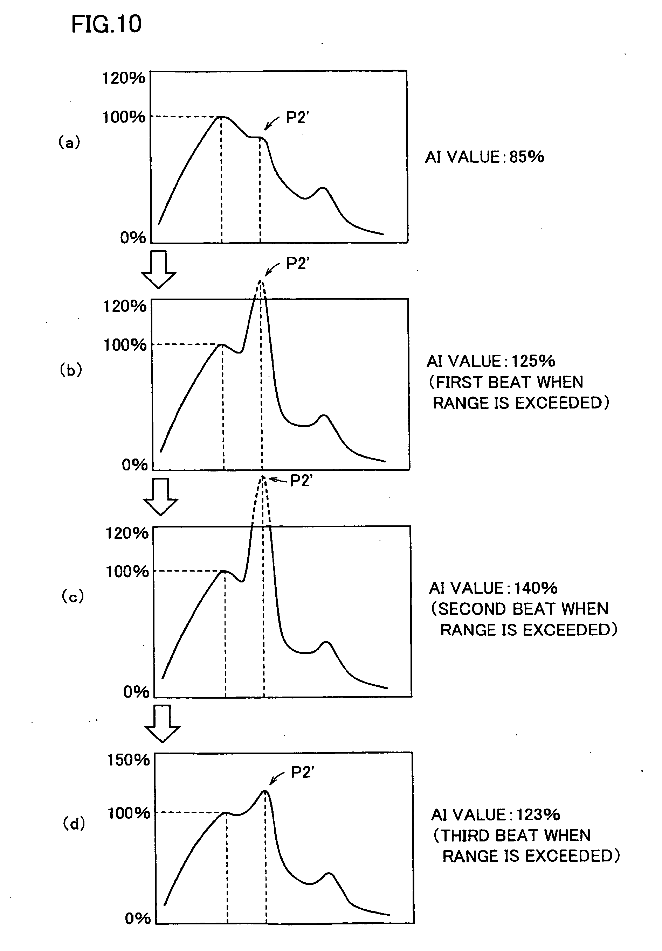

Fig. 10 will now be described with reference to the flowchart in Fig. 9. Fig. 10 shows a specific example in the second embodiment of the present invention. Here, the upper limit value of the vertical range is adjusted. Each of Figs. 10(a)-(d) shows an example of how a waveform of one beat, which has been cut out successively, is displayed. Here, the examples are described as the a-th to d-th waveforms.

Initially, assume that the vertical range stored in RAM 25 has an upper limit value of 120%. Furthermore, assume that the a-th waveform has an AI value of 85%, the b-th waveform has an AI value of 125%, the c-th waveform has an AI value of 140%, and the d-th waveform has an AI value of 123%. Therefore, in this case, the values at reflected wave peak points P2' on the ordinate axis in the normalized waveform f(x) calculated in step S204 in Fig. 8 are also 85%, 125%, 140%, and 123%, respectively.

Referring to Fig. 10(a), the value at reflected wave peak point P2' on the ordinate axis in the a-th waveform, in other words, the AI value, is 85%. Since this value falls within the upper limit value of the vertical range, namely, 120% (NO in S1103), CPU 23 plots the waveform lying within the upper limit value of 120% in steps S208'-S212'. Therefore a user can grasp the position (height) of reflected wave peak point P2' on the screen.

Then, referring to Fig. 10(b), the value at reflected wave peak point P2' on the ordinate axis in the b-th waveform, in other words, the AI value, is 125%. Since this value exceeds the upper limit value of the vertical range, namely, 120% (YES in S1103), count number N is set to "1" (S1104). While a count number is set to "1", the upper limit value of the vertical range is not adjusted. Therefore, a part of the waveform within the upper limit value of 120% is plotted in step S208', and thus another part of the waveform shown with a dotted line in Fig. 10(b) is not displayed, resulting in that the position of reflected wave peak point P2' cannot be viewed.

Then, referring to Fig. 10(c), the value at reflected wave peak point P2' on the ordinate axis in the c-th waveform, in other words, the AI value, is 140%. Since this value exceeds the upper limit value of the vertical range (YES in S 1103), the count number is set to "2" (S1104). In this case, the count number has not reached "3", and thus a part of the waveform within the upper limit value of 120% is plotted in step S208'. In this case, a part of the waveform shown with a dotted line in the drawing is not displayed either, resulting in that the position of reflected wave peak point P2' cannot be viewed.

Then, referring to Fig. 10(d), the value at reflected wave peak point P2' on the ordinate axis in the d-th waveform, in other words, the AI value, is 123%. Since this value exceeds the upper limit value of the vertical range (YES in S1103), the count number is set to "3" (S1104). Here, the count number has reached "3" (YES in S1106), and thus the upper limit value of the vertical range is adjusted (S 1108). Specifically, since the maximum value of three reflected wave peak points P2' in the waveforms corresponding to the three beats counted (the b-th to d-th waveforms) is found to be 140% in the c-th waveform, an upper limit value of 150%, which equals to the maximum plus 10%, is set as an updated upper limit value. In this case, the waveform within the upper limit value of 150% is plotted in step S208', and thus the position of reflected wave peak point P2' (position at 123%) can be viewed.

As such, in the second embodiment, if the value at the reflected wave peak point exceeds the upper limit value of the vertical range three times in succession, the upper limit value is updated. Thereby, it is possible to eliminate such an inconvenience that the position (height) of the reflected wave peak point is not displayed for a long period or time. Therefore, even if a subject has an unusually high reflected wave peak point, the AI value can appropriately be grasped

Since the upper limit value is allowed to be adjusted only when it is exceeded by the value at the reflected wave peak point three times in succession, it is possible to eliminate such an inconvenience that the vertical range is frequently adjusted owing to a noise or the like, unwantedly making it difficult to detect the change in AI value. In the present embodiment, the upper limit value is updated when the value at the reflected wave peak point exceeds the vertical range three times in succession. However, how update is performed is not limited thereto. For example, the upper limit value may be updated when the value at the reflected wave peak point exceeds the vertical range two out of ten times, or may simply be updated when the value at the reflected wave peak point exceeds the vertical range once.

In the second embodiment, determination as to whether or not a value (ratio) at the reflected wave peak point in the normalized waveform exceeds the vertical range. Alternatively, the determination may be made with a value (ratio) at the global maximum in the normalized waveform.

Since the value at the reflected wave peak point in the normalized waveform is usually equal to the AI value, the determination above may be made with the AI value. In this case, the vertical range may be adjusted before the original waveform is normalized.

The above-described method of displaying pulse wave information by the pulse wave information display apparatus according to the present invention may be provided in the form of a program. Such a program may be provided as a program product by being recorded on such a computer-readable recording medium as a flexible disk, a Compact Disc-ROM (CD-ROM), a ROM, and a RAM, and a memory card, which are all attached to a computer. Alternatively, the program may be provided by being recorded on such a recording medium as a hard disk included in a computer. Moreover, the program may be provided by being downloaded via a network.

The program product thus provided is executed by being installed in such a program storage unit as a hard disk. The program product includes a program itself and a recording medium with the program recorded thereon.

Although the present invention has been described and illustrated in detail, it is clearly understood that the same is by way of illustration and example only and is not to be taken by way of limitation, the scope of the present invention being limited only by the terms of the appended claims.

## Claims

1.    A pulse wave information display apparatus, comprising:

>    a storage portion (25) for storing measured pulse wave data;
>    an extraction portion (23, S116) for extracting a waveform of one beat from said stored pulse wave data;
>    **characterized by**
>    a specification portion (23, S118) for specifying a first characteristic point corresponding to a global maximum of an early systolic component in said extracted waveform of one beat;
>    a normalization portion (23, S204) for normalizing said waveform of one beat based on an amplitude value at said specified first characteristic point;
>    a plot portion (23, S208) for generating a signal for plotting said normalized waveform of one beat; and
>    a display portion (22) for displaying said normalized waveform of one beat based on an output from said plot portion.

**2.** The pulse wave information display apparatus according to claim 1, wherein said specification portion further specifies a second characteristic point corresponding to a global maximum of a late systolic component in said extracted waveform of one beat,

the pulse wave information display apparatus further comprising a marker display portion (23, S212) for displaying markers on said display portion to indicate a position corresponding to said first characteristic point and a position corresponding to said second characteristic point in said normalized waveform of one beat.

**3.** The pulse wave information display apparatus according to claim 2, wherein said marker display portion further displays markers on said display portion to indicate a position corresponding to a pulse wave rising point in said normalized waveform.

**4.** The pulse wave information display apparatus according to claim 1, further comprising a pulse wave measurement portion (1) for measuring said pulse wave data.

**5.** The pulse wave information display apparatus according to claim 1, wherein said specification portion further specifies a second characteristic point corresponding to a global maximum of a late systolic component in said extracted waveform of one beat,

the pulse wave information display apparatus further comprising a ratio display portion (23, S214) for simultaneously displaying on said display portion a ratio of an amplitude value at said second characteristic point to an amplitude value at said first characteristic point, and said normalized waveform of one beat.

**6.** The pulse wave information display apparatus according to claim 1, displaying on said display portion a waveform based on a user's instruction and said normalized waveform in a superimposed manner.

**7.** The pulse wave information display apparatus according to claim 1, wherein said specification portion further specifies a second characteristic point corresponding to a global maximum of a late systolic component an said extracted waveform of one beat,

the pulse wave information display apparatus further comprising

a determination portion (23, S1103) for determining whether or not a value at a point corresponding to said second characteristic point in said normalized waveform exceeds a displayable upper limit value, and

an update portion (23, S1108) for updating said upper limit value based on a result of the determination by said determination portion such that the value at the point corresponding to said second characteristic point can be displayed.

**8.** The pulse wave information display apparatus according to claim 7, wherein when said value at the point corresponding to said second characteristic point exceeds said upper limit value a predetermined number of times, said update portion updates said upper limit value by using a maximum value among the points, each corresponding to said second characteristic point, obtained said predetermined number of times.

**9.** A computer-readable medium on which a program is stored, for controlling a pulse wave information display apparatus (2) including a storage portion (25) for storing pulse wave data, an arithmetic processing portion (23) for arithmetically processing the pulse wave data stored in said storage portion, and a display portion (22), allowing the arithmetic processing portion to perform the steps of:

extracting a waveform of one beat from the pulse wave data stored in said storage portion (S116);
specifying a first characteristic point corresponding to a global maximum of an early systolic component in said extracted waveform of one beat (S118);
normalizing said waveform of one beat based on an amplitude value at said specified first characteristic point (S204); and
displaying said normalized waveform of one beat on said display portion (S208).

**10.** The computer-readable medium according to claim 9, wherein said step of specifying includes the step of specifying a second characteristic point corresponding to a global maximum of a late systolic component in said extracted waveform of one beat,

the computer-readable medium further allowing said arithmetic processing portion to perform the step of displaying markers on said display portion to indicate a position corresponding to said first characteristic point and a position corresponding to said second characteristic point in said normalized waveform of one beat (S212).

**11.** The computer-readable medium according to claim 9, wherein said step of specifying includes the step of specifying a second characteristic point corresponding to a global maximum of a late systolic component in said extracted waveform of one beat,

the computer-readable medium further allowing said arithmetic processing portion to perform the step of simultaneously displaying on said display portion a ratio of an amplitude value at said second characteristic point to an amplitude value at said first characteristic point, and said normalized waveform of one beat (S214).

**12.** The computer-readable medium according to claim 9, wherein said step of specifying includes the step of specifying a second characteristic point corresponding to a global maximum of a late systolic component in said extracted waveform of one beat,

the computer-readable medium further allowing said arithmetic processing portion to perform the steps of

determining whether or not a value at a point corresponding to said second characteristic point in said normalized waveform exceeds a displayable upper limit value (S1103), and

updating said upper limit value based on a result of the determination such that the value at the point corresponding to said second characteristic point can be displayed (S1108).

**13.** A method of displaying pulse wave information in a pulse wave information display apparatus including a storage portion (25) for storing pulse wave data, and a display portion (22) for displaying information based on said pulse wave data, comprising the steps of:

extracting a waveform of one beat from the pulse wave data stored in said storage portion (S116); **characterized by**

specifying a first characteristic point corresponding to a global maximum of an early systolic component in said extracted waveform of one beat (S118);

normalizing said waveform of one beat based on an amplitude value at said specified first characteristic point (S204); and

displaying said normalized waveform of one beat on said display portion (S208).

**14.** The method of displaying pulse wave information according to claim 13, wherein said step of specifying includes the step of specifying a second characteristic point corresponding to a global maximum of late systolic component in said extracted waveform of one beat,

the method further comprising the step of displaying markers on said display portion to indicate a position corresponding to said first characteristic point and a position corresponding to said second characteristic point in said normalized waveform of one beat (S212).

**15.** The method of displaying pulse wave information according to claim 13, wherein said step of specifying includes the step of specifying a second characteristic point corresponding to a global maximum of late systolic component in said extracted waveform of one beat,

the method further comprising the step of simultaneously displaying on said display portion a ration of an amplitude value at said second characteristic point to an amplitude value at said first characteristic point, and said normalized waveform of one beat (S214).

**16.** The method of displaying pulse wave information according to claim 13, wherein said step of specifying includes the step of specifying a second characteristic point corresponding to a global maximum of a late systolic component in said extracted waveform of one beat,

the method further comprising the steps of

determining whether or not a value at a point corresponding to said second characteristic point in said normalized waveform exceeds a displayable upper limit value (S1103), and

updating said upper limit value based on a result of the determination such that the value at the point corresponding to said second characteristic point can be displayed (S1108).

**Patentansprüche**

**1.** Pulswelleninformationsanzeigevorrichtung, welche aufweist:

einen Speicherabschnitt (25) zur Speicherung gemessener Pulswellendaten;
einen Extraktionsabschnitt (23, S116) zum Extrahieren einer Wellenform eines Herzschlages aus den gespeicherten Pulswellendaten; **gekennzeichnet durch**

einen Spezifikationsabschnitt (23, S118) zur Spezifizierung eines ersten Kennpunktes, der einem globalen Maximum einer frühen systolischen Komponente in der extrahierten Wellenform eines Herzschlags entspricht;

einen Normierungsabschnitt (23, S204) zur Normierung der Wellenform eines Herzschlags beruhend auf einem Amplitudenwert an dem spezifizierten ersten Kennpunkt;

einen Auftragabschnitt (23, S208) zur Erzeugung eines Signals zum Auftragen der normierten Wellenform eines Herzschlags; und

einen Anzeigeabschnitt (22) zur Anzeige der normierten Wellenform eines Herzschlags beruhend auf einer Ausgabe des Auftragabschnitts.

2. Pulswelleninformationsanzeigevorrichtung nach Anspruch 1, wobei der Spezifikationsabschnitt ferner einen zweiten Kennpunkt, der einem globalen Maximum einer späten systolischen Komponente in der extrahierten Wellenform eines Herzschlags entspricht, spezifiziert,

wobei die Pulswelleninformationsanzeigevorrichtung ferner einen Markierungsanzeigeabschnitt (23, S212) zur Anzeige von Markierungen auf dem Anzeigeabschnitt zur Angabe einer Position, die dem ersten Kennpunkt in der normierten Wellenform eines Herzschlags entspricht, und einer Position die dem zweiten Kennpunkt in der normierten Wellenform eines Herzschlags entspricht, aufweist.

3. Pulswelleninformationsanzeigevorrichtung nach Anspruch 2, wobei der Markierungsanzeigeabschnitt ferner Markierungen auf dem Anzeigeabschnitt zur Angabe einer Position, die einem Pulswellenanstiegspunkt in der normierten Wellenform entspricht, anzeigt.

4. Pulswelleninformationsanzeigevorrichtung nach Anspruch 1, welche ferner einen Pulswellenmessabschnitt (1) zur Messung der Pulswellendaten aufweist.

5. Pulswelleninformationsanzeigevorrichtung nach Anspruch 1, wobei der Spezifikationsabschnitt ferner einen zweiten Kennpunkt, der einem globalen Maximum einer späten systolischen Komponente in der extrahierten Wellenform eines Herzschlags entspricht, spezifiziert,

wobei die Pulswelleninformationsanzeigevorrichtung ferner einen Verhältnisanzeigeabschnitt (23, S214) zur gleichzeitigen Anzeige eines Verhältnisses eines Amplitudenwerts an dem zweiten Kennpunkt zu einem Amplitudenwert an dem ersten Kennpunkt und der normierten Wellenform eines Herzschlags auf dem Anzeigeabschnitt aufweist.

6. Pulswelleninformationsanzeigevorrichtung nach Anspruch 1, welche auf dem Anzeigeabschnitt eine auf einem Anwenderbefehl beruhende Wellenform und die normierte Wellenform in überlagerter Weise anzeigt.

7. Pulswelleninformationsanzeigevorrichtung nach Anspruch 1, wobei der Spezifikationsabschnitt ferner einen zweiten Kennpunkt spezifiziert, der einem globalen Maximum einer späten systolischen Komponente in der extrahierten Wellenform eines Herzschlags entspricht,

wobei die Pulswelleninformationsanzeigevorrichtung ferner

einen Bestimmungsabschnitt (23, S1103) zur Bestimmung, ob ein Wert an einem Punkt, der dem zweiten Kennpunkt in der normierten Wellenform entspricht, einen anzeigbaren oberen Grenzwert überschreitet oder nicht, und

einen Aktualisierungsabschnitt (23, S1108) zur Aktualisierung des oberen Grenzwerts beruhend auf einem Ergebnis der Bestimmung mit dem Bestimmungsabschnitt, derart, dass der Wert an dem Punkt, der dem zweiten Kennpunkt entspricht, angezeigt werden kann, aufweist.

8. Pulswelleninformationsanzeigevorrichtung nach Anspruch 7, wobei, wenn der Wert an dem Punkt, der dem zweiten Kennpunkt entspricht, den oberen Grenzwert eine bestimmte Anzahl von Malen überschreitet, der Aktualisierungsabschnitt den oberen Grenzwert unter Verwendung eines Maximalwerts unter den die bestimmte Anzahl von Malen gewonnenen Punkten, von denen jeder dem zweiten Kennpunkt entspricht, aktualisiert.

9. Computerlesbares Medium, auf welchem ein Programm gespeichert ist, zur Steuerung einer Pulswelleninformationsanzeigevorrichtung (2), welche einen Speicherabschnitt (25) zur Speicherung von Pulswellendaten, einen Rechenverarbeitungsabschnitt (23) zur rechnerischen Verarbeitung der in dem Speicherabschnitt gespeicherten Pulswellendaten und einen Anzeigeabschnitt (22) enthält, womit der Rechenverarbeitungsabschnitt folgende Schritte durchführen kann:

Extrahieren einer Wellenform eines Herzschlages aus den in dem Speicherabschnitt gespeicherten Pulswellendaten (S116);

Spezifizieren eines ersten Kennpunkts, der einem globalen Maximum einer frühen systolischen Komponente in der extrahierten Wellenform eines Herzschlags entspricht (S118);
Normieren der Wellenform eines Herzschlags beruhend auf einem Amplitudenwert an dem spezifizierten ersten Kennpunkt (S204); und
Anzeigen der normierten Wellenform eines Herzschlags auf dem Anzeigeabschnitt (S208).

10. Computerlesbares Medium nach Anspruch 9, wobei der Spezifizierungsschritt den Schritt des Spezifizierens eines zweiten Kennpunkts, der einem globalen Maximum einer späten systolischen Komponente in der extrahierten Wellenform eines Herzschlags entspricht, enthält,
wobei das computerlesbare Medium ferner ermöglicht, dass der Rechenverarbeitungsabschnitt den Schritt des Anzeigens von Markierungen auf dem Anzeigeabschnitt zur Angabe einer Position, die dem ersten Kennpunkt in der normierten Wellenform eines Herzschlags entspricht, und einer Position, die dem zweiten Kennpunkt der normierten Wellenform eines Herzschlags entspricht, durchführen kann (S212).

11. Computerlesbares Medium nach Anspruch 9, wobei der Schritt des Spezifizierens den Schritt des Spezifizerens eines zweiten Kennpunkts, der einem globalen Maximum einer späten systolischen Komponente in der extrahierten Wellenform eines Herzschlags entspricht, enthält,
wobei das computerlesbare Medium ferner ermöglicht, dass der Rechenverarbeitungsabschnitt den Schritt des gleichzeitigen Anzeigens, auf dem Anzeigeabschnitt, eines Verhältnisses eines Amplitudenwerts an dem zweiten Kennpunkt zu einem Amplitudenwert an dem ersten Kennpunkt und der normierten Wellenform eines Herzschlags durchführen kann (S214).

12. Computerlesbares Medium nach Anspruch 9, wobei der Schritt des Spezifizierens den Schritt des Spezifizierens eines zweiten Kennpunkts, der einem globalen Maximum einer späten systolischen Komponente in der extrahierten Wellenform eines Herzschlags entspricht, enthält,
wobei das computerlesbare Medium ferner ermöglicht, dass der Rechenverarbeitungsabschnitt die Schritte des Bestimmens, ob ein Wert an einem Punkt, der dem zweiten Kennpunkt in der normierten Wellenform entspricht, einen anzeigbaren oberen Grenzwert überschreitet oder nicht (S1103), und
Aktualisierens des oberen Grenzwerts, beruhend auf einem Ergebnis der Bestimmung, derart, dass der Wert an dem Punkt, der dem zweiten Kennpunkt entspricht, angezeigt werden kann (S1108), durchführen kann.

13. Verfahren der Anzeige von Pulswelleninformation in einer Pulswelleninformationsanzeigevorrichtung, welche einen Speicherabschnitt (25) zur Speicherung von Pulswellendaten und einen Anzeigeabschnitt (22) zur Anzeige von Information beruhend auf den Pulswellendaten enthält, wobei das Verfahren folgende Schritte aufweist:

Extrahieren einer Wellenform eines Herzschlags aus den in dem Speicherabschnitt gespeicherten Pulswellendaten (S116); **gekennzeichnet durch**
Spezifizieren eines ersten Kennpunkts, der einem globalen Maximum einer frühen systolischen Komponente in der extrahierten Wellenform eines Herzschlags entspricht (S118);
Normieren der Wellenform eines Herzschlags beruhend auf einem Amplitudenwert an dem spezifizierten Kennpunkt (S204); und
Anzeigen der normierten Wellenform eines Herzschlags auf dem Anzeigeabschnitt (S208).

14. Verfahren der Anzeige von Pulswelleninformation gemäß Anspruch 13, wobei der Schritt des Spezifizierens den Schritt des Spezifizierens eines zweiten Kennpunkts, der einem globalen Maximum einer späten systolischen Komponente in der extrahierten Wellenform eines Herzschlags entspricht, enthält,
wobei das Verfahren ferner den Schritt des Anzeigens von Markierungen auf dem Anzeigeabschnitt zur Angabe einer Position, die dem ersten Kennpunkt in der normierten Wellenform eines Herzschlags entspricht, und einer Position, die dem zweiten Kennpunkt in der normierten Wellenform eines Herzschlags entspricht, aufweist (S212).

15. Verfahren der Anzeige von Pulswelleninformation nach Anspruch 13, wobei der Schritt des Spezifizierens den Schritt des Spezifizierens eines zweiten Kennpunkts, der einem globalen Maximum einer späten systolischen Komponente in der extrahierten Wellenform eines Herzschlags entspricht, enthält,
wobei das Verfahren ferner den Schritt des gleichzeitigen Anzeigens, auf dem Anzeigeabschnitt, eines Verhältnisses eines Amplitudenwerts an dem zweiten Kennpunkt zu einem Amplitudenwert am ersten Kennpunkt und der normierten Wellenform eines Herzschlags aufweist (S214).

16. Verfahren der Anzeige von Pulswelleninformation nach Anspruch 13, wobei der Schritt des Spezifizierens den Schritt des Spezifizierens eines zweiten Kennpunkts, der einem globalen Maximum einer späten systolischen Komponente in der extrahierten Wellenform eines Herzschlags entspricht, enthält,
wobei das Verfahren ferner die Schritte des
Bestimmens, ob ein Wert an einem Punkt, der dem zweiten Kennpunkt in der normierten Wellenform entspricht, einen anzeigbaren oberen Grenzwert überschreitet oder nicht (S1103), und
Aktualisierens des oberen Grenzwerts beruhend auf einem Ergebnis der Bestimmung, derart, dass der Wert an dem Punkt, der dem zweiten Kennpunkt entspricht, angezeigt werden kann (S1108), aufweist.

**Revendications**

1.  Dispositif d'affichage d'informations d'onde de pouls, comprenant :

    ♦ une partie de stockage (25) destinée à stocker des données d'onde de pouls mesurées;
    ♦ une partie d'extraction (23, S116) destinée à extraire une forme d'onde d'un battement desdites données d'onde de pouls stockées ; **caractérisé en ce qu'**il comprend

    ♦ une partie de spécification (23, S118) destinée à spécifier un premier point caractéristique correspondant à un maximum global d'un élément systolique précoce dans ladite forme d'onde d'un battement extraite;
    ♦ une partie de normalisation (23, S204) destinée à normaliser ladite forme d'onde d'un battement sur la base d'une valeur de grandeur au niveau dudit premier point caractéristique spécifié ;
    ♦ une partie de tracé (23, S208) destinée à générer un signal pour le traçage de ladite forme d'onde d'un battement normalisée ; et
    ♦ une partie d'affichage (22) destinée à afficher ladite forme d'onde d'un battement normalisé sur la base d'une production depuis ladite partie de tracé.

2.  Dispositif d'affichage d'informations d'onde de pouls selon la revendication 1, dans lequel ladite partie de spécification spécifie en outre un second point caractéristique correspondant à un maximum global d'un élément systolique tardif dans ladite forme d'onde d'un battement extraite, le dispositif d'affichage d'informations d'onde de pouls comprenant en outre une partie d'affichage de marqueurs (23, S212) destinée à afficher des marqueurs sur ladite partie d'affichage afin d'indiquer une position correspondant audit premier point caractéristique et une position correspondant audit second point caractéristique dans ladite forme d'onde d'un battement normalisée.

3.  Dispositif d'affichage d'informations d'onde de pouls selon la revendication 2, dans lequel ladite partie d'affichage de marqueurs affiche en outre des marqueurs sur ladite partie d'affichage afin d'indiquer une position correspondant à un point d'augmentation de l'onde de pouls dans ladite forme d'onde normalisée.

4.  Dispositif d'affichage d'informations d'onde de pouls selon la revendication 1, comprenant en outre une partie de mesure d'onde de pouls (1) destinée à mesurer lesdites données d'onde de pouls.

5.  Dispositif d'affichage d'informations d'onde de pouls selon la revendication 1, dans lequel ladite partie de spécification spécifie en outre un second point caractéristique correspondant à un maximum global d'un élément systolique tardif dans ladite forme d'onde d'un battement extraite, le dispositif d'affichage d'informations d'onde de pouls comprenant en outre une partie d'affichage de rapport (23, S214) destinée à afficher simultanément sur ladite partie d'affichage un rapport d'une valeur de grandeur au niveau dudit second point caractéristique à une valeur de grandeur au niveau dudit premier point caractéristique, et ladite forme d'onde d'un battement normalisée.

6.  Dispositif d'affichage d'informations d'onde de pouls selon la revendication 1, affichant sur ladite partie d'affichage une forme d'onde basée sur les instructions d'un utilisateur et ladite forme d'onde étant normalisée de manière superposée.

7.  Dispositif d'affichage d'informations d'onde de pouls selon la revendication 1, dans lequel ladite partie de spécification spécifie en outre un second point caractéristique correspondant à un maximum global d'un élément systolique tardif sur ladite forme d'onde d'un battement extraite,
le dispositif d'affichage d'informations d'onde de pouls comprenant en outre
une partie de détermination (23, S1103) destinée à déterminer si une valeur à un point correspondant audit second point caractéristique dans ladite forme d'onde normalisée dépasse ou non une valeur de limite supérieure affichable, et

une partie de mise à jour (23, S1108) destinée à mettre à jour ladite valeur de limite supérieure sur la base d'un résultat de la détermination par ladite partie de détermination de sorte que la valeur au point correspondant audit second point caractéristique puisse être affichée.

8. Dispositif d'affichage d'informations d'onde de pouls selon la revendication 7, dans lequel lorsque ladite valeur au point correspondant audit second point caractéristique dépasse ladite valeur de limite supérieure un nombre prédéterminé de fois, ladite partie de mise à jour met à jour ladite valeur de limite supérieure en utilisant une valeur maximum parmi les points, chacun correspondant audit second point caractéristique, obtenus ledit nombre de fois prédéterminé.

9. Support informatique sur lequel un programme est stocké, destiné à commander un dispositif d'affichage d'informations d'onde de pouls (2) comprenant une partie de stockage (25) destinée à stocker des données d'onde de pouls, une partie de traitement arithmétique (23) destinée à traiter de manière arithmétique les données d'onde de pouls stockées dans ladite partie de stockage, et une partie d'affichage (22) permettant à la partie de traitement arithmétique de réaliser les étapes consistant à :

♦ extraire une forme d'onde d'un battement à partir des données d'onde de pouls stockées dans ladite partie de stockage (S116) ;
♦ spécifier un premier point caractéristique correspondant à un maximum global d'un élément systolique précoce dans ladite forme d'onde d'un battement extraite (S118) ;
♦ normaliser ladite forme d'onde d'un battement sur la base d'une valeur de grandeur au niveau dudit premier point caractéristique spécifié (S204) ; et
♦ afficher ladite forme d'onde d'un battement normalisée sur ladite partie d' affichage (S208).

10. Support informatique selon la revendication 9, dans lequel ladite étape de spécification comprend l'étape consistant à spécifier un second point caractéristique correspondant à un maximum global d'un élément systolique tardif dans ladite forme d'onde d'un battement extraite, le support informatique permettant en outre à ladite partie de traitement arithmétique de réaliser l'étape d'affichage de marqueurs sur ladite partie d'affichage afin d'indiquer une position correspondant audit premier point caractéristique et une position correspondant audit second point caractéristique dans ladite forme d'onde d'un battement normalisée (S212).

11. Support informatique selon la revendication 9, dans lequel ladite étape de spécification comprend l'étape consistant à spécifier un second point caractéristique correspondant à un maximum global d'un élément systolique tardif dans ladite forme d'onde d'un battement extraite, le support informatique permettant en outre à ladite partie de traitement arithmétique de réaliser l'étape consistant à afficher simultanément sur ladite partie d'affichage un rapport d'une valeur de grandeur audit second point caractéristique à une valeur de grandeur audit premier point caractéristique, et à ladite forme d'onde d'un battement normalisée (S214).

12. Support informatique selon la revendication 9, dans lequel ladite étape de spécification comprend l'étape consistant à spécifier un second point caractéristique correspondant à un maximum global d'un élément systolique tardif dans ladite forme d'onde d'un battement extraite,
le support informatique permettant en outre à ladite partie de traitement arithmétique de réaliser les étapes consistant à
déterminer si une valeur au niveau du point correspondant audit second point caractéristique dans ladite forme d'onde normalisée dépasse ou non une valeur de limite supérieure affichable (S1103), et
mettre à jour de ladite valeur de limite supérieure sur la base d'un résultat de la détermination de sorte que la valeur au niveau du point correspondant audit second point caractéristique puisse être affichée (S1108).

13. Procédé d'affichage d'informations d'onde de pouls sur un dispositif d'affichage d'informations d'onde de pouls comprenant une partie de stockage (25) destinée à stocker des données d'onde de pouls, et une partie d'affichage (22) destinée à afficher des informations basées sur lesdites données d'onde de pouls, comprenant les étapes consistant à :

extraire une forme d'onde d'un battement à partir des données d'onde de pouls stockées ladite partie de stockage (S116) ; **caractérisé par** le fait de
spécifier un premier point caractéristique correspondant à un maximum global d'un élément systolique précoce dans ladite forme d'onde d'un battement extraite (S118) ;
normaliser ladite forme d'onde d'un battement sur la base d'une valeur de grandeur au niveau dudit premier point caractéristique spécifié (3204); et

afficher ladite forme d'onde d'un battement normalisée sur ladite partie d'affichage (S208).

14. Procédé d'affichage d'informations d'onde de pouls selon la revendication 13, dans lequel ladite étape de spécification comprend l'étape consistant à spécifier un second point caractéristique correspondant à un maximum global d'un élément systolique tardif dans ladite forme d'onde d'un battement extraite, le procédé comprenant en outre l'étape consistant à afficher des marqueurs sur ladite partie d'affichage afin d'indiquer une position correspondant audit premier point caractéristique et une position correspondant audit second point caractéristique dans ladite forme d'onde d'un battement normalisée (S212).

15. Procédé d'affichage d'informations d'onde de pouls selon la revendication 13, dans lequel ladite étape de spécification comprend l'étape consistant à spécifier un second point caractéristique correspondant à un maximum global d'un élément systolique tardif dans ladite forme d'onde d'un battement extraite, le procédé comprenant en outre l'étape consistant à afficher simultanément sur ladite partie d'affichage un rapport d'une valeur de grandeur audit second point caractéristique à une valeur de grandeur audit premier point caractéristique, et à ladite forme d'onde d'un battement normalisée (S214).

16. Procédé d'affichage d'informations d'onde de pouls selon la revendication 13, dans lequel ladite étape de spécification comprend l'étape consistant à spécifier un second point caractéristiqué correspondant à un maximum global d'un élément systolique tardif dans ladite forme d'onde d'un battement extraite,

le procédé comprenant en outre les étapes consistant à

déterminer si une valeur au niveau du point correspondant audit second point caractéristique dans ladite forme d'onde normalisée dépasse ou non une valeur de limite supérieure affichable (S1103), et

mettre à jour ladite valeur de limite supérieure sur la base d'un résultat de la détermination de sorte que la valeur au niveau du point correspondant audit second point caractéristique puisse être affichée (S1108).

## FIG.1

## FIG.2

```
                    ┌──────────────┐
                    │    START     │
                    └──────┬───────┘
                           │        ┌─ S102
                    ┌──────▼───────┐
                    │    i←0       │
                    └──────┬───────┘
     ┌─────────────────────┤        ┌─ S104
     │              ┌──────▼────────────────┐
     │              │ OBTAIN PULSE WAVE DATA │
     │              └──────┬────────────────┘
     │                     │        ┌─ S106
     │              ┌──────▼────────────────┐
     │              │ OBTAIN N-TH DERIVATIVE │
     │              └──────┬────────────────┘
     │                     │        ┌─ S108
     │              ┌──────▼────────┐
     │              │ DIVIDE PULSE WAVE │
     │              └──────┬────────┘
     │                     │        ┌─ S114
     │              ┌──────▼────────┐
     │              │    i←i+1      │
     │              └──────┬────────┘
     │                     │        ┌─ S116
     │          ┌──────────▼────────────────┐
     │          │ CUT OUT THE i-TH WAVEFORM  │
     │          └──────────┬────────────────┘
     │                     │        ┌─ S118
     │       ┌─────────────▼──────────────┐
     │       │ EXTRACT CHARACTERISTIC POINT │
     │       └─────────────┬──────────────┘
     │                     │        ┌─ S120
     │              ┌──────▼────────┐
     │              │  CALCULATE AI  │
     │              └──────┬────────┘
     │                     │        ┌─ S122
     │           ┌─────────▼─────────┐
     │           │ DISPLAY PROCESSING │
     │           └─────────┬─────────┘
     │                     │        ┌─ S124
     │        YES  ╱───────▼────────╲
     └────────────┤    IS NEXT       │
                  │  DATA PRESENT     │
                  ╲        ?         ╱
                   ╲──────┬─────────╱
                          │ NO
                    ┌─────▼────────┐
                    │     END      │
                    └──────────────┘
```

FIG.3

B1

2002/06/15
10:34:57
B8

SYS
    132mmHg
DIA
    74mmHg
B7

B3          B2

AI

HR
    74bpm
B6

HR

B4

AI
85%
B5

EP 1 645 225 B1

FIG.4

AI VALUE : 85%

(a)

AI VALUE : 110%

(b)

FIG.5

## FIG.6

```
          ( START DISPLAY PROCESSING )
                        │
                        ▼          ┌─ S204
        │ CALCULATE THE i-TH WAVEFORM f(x) │
                        │
                        ▼          ┌─ S206
      │ CLEAR PLOT AS TO THE PREVIOUS WAVEFORM │
                        │
                        ▼          ┌─ S208
            │ PLOT WAVEFORM f(x) │
                        │
                        ▼          ┌─ S210
              │ PLOT BASELINE │
                        │
                        ▼          ┌─ S212
  │ PLOT PERPENDICULAR LINES FROM EJECTED │
  │ WAVE PEAK POINT TO BASELINE AND FROM   │
  │ REFLECTED WAVE PEAK POINT TO BASELINE  │
                        │
                        ▼          ┌─ S214
            │ DISPLAY AI VALUE │
                        │
                        ▼
              ( RETURN )
```

FIG.7

# FIG.8

```
        ( START DISPLAY PROCESSING )
                    │
                    ▼        ┌─ S204
        ┌─────────────────────────────────────┐
        │  CALCULATE THE i-TH WAVEFORM f(x)   │
        └─────────────────────────────────────┘
                    │        ┌─ S205
                    ▼
        ┌─────────────────────────────────────┐
        │      ADJUST VERTICAL RANGE          │
        └─────────────────────────────────────┘
                    │              ┌─ S206
                    ▼
        ┌─────────────────────────────────────────┐
        │  CLEAR PLOT AS TO THE PREVIOUS WAVEFORM  │
        └─────────────────────────────────────────┘
                    │        ┌─ S208'
                    ▼
        ┌─────────────────────────────────────┐
        │          PLOT WAVEFORM f(x)         │
        └─────────────────────────────────────┘
                    │        ┌─ S210'
                    ▼
        ┌─────────────────────────────────────┐
        │            PLOT BASELINE            │
        └─────────────────────────────────────┘
                    │              ┌─ S212'
                    ▼
        ┌─────────────────────────────────────────┐
        │  PLOT PERPENDICULAR LINES FROM EJECTED  │
        │  WAVE PEAK POINT TO BASELINE AND FROM   │
        │  REFLECTED WAVE PEAK POINT TO BASELINE  │
        └─────────────────────────────────────────┘
                    │        ┌─ S214
                    ▼
        ┌─────────────────────────────────────┐
        │          DISPLAY AI VALUE           │
        └─────────────────────────────────────┘
                    │
                    ▼
            (        RETURN        )
```

## FIG.9

START ADJUSTING VERTICAL RANGE

S1101
i=1 ? — NO
YES

S1102
N←0

S1103
DOES THE i-TH WAVEFORM EXCEED VERTICAL RANGE? — NO
YES

S1104
N←N+1

S1105
STORE VALUE (RATIO) AT REFLECTED WAVE PEAK POINT

S1106
N=3 ? — NO
YES

S1108
THE UPPER LIMIT OF VERTICAL DISPLAY RANGE←
THE MAXIMUM IN PULSE WAVES OF PREVIOUS THREE BEATS+10%

S1110
N←0

RETURN

## FIG.10

(a)

AI VALUE : 85%

(b)

AI VALUE : 125%
(FIRST BEAT WHEN
RANGE IS EXCEEDED)

(c)

AI VALUE : 140%
(SECOND BEAT WHEN
RANGE IS EXCEEDED)

(d)

AI VALUE : 123%
(THIRD BEAT WHEN
RANGE IS EXCEEDED)

## FIG.11

AMPLITUDE VALUE
OF PULSE WAVE

## FIG.12

AMPLITUDE VALUE
OF PULSE WAVE

FIG.13

ORIGINAL WAVEFORM

FIRST

SECOND

PP

PQ

PA

PB

DP

DZC

APG-A

APG-C

APG-E

EP 1 645 225 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 7039530 A
- US 5265011 A
- JP 2004195204 A
- EP 1426007 A